# EUROPEAN PATENT APPLICATION

(11) **EP 2 238 969 A1**
(43) Date of publication of application: **13.10.2010**
(21) Application number: 08866144.2
(22) Date of filing: 19.12.2008
(51) Int. Cl.: A61K 8/44, A61K 31/221, A61P 3/04, A61P 17/00, A61P 17/08, A61P 17/14, A61Q 19/00

(54) **PREPARATION FOR EXTERNAL APPLICATION TO SKIN AND COSMETIC PREPARATION**

(30) Priority: 28.12.2007 JP 2007339827
(71) Applicant: Showa Denko K.K., Tokyo 105-8518 (JP)
(72) Inventor: AOKI, Hirobumi, Chiba-shi Chiba 267-0056 (JP); KAMACHI, Harumi, Chiba-shi Chiba 267-0056 (JP)
(74) Representative: Strehl Schübel-Hopf & Partner
(86) International application number: PCT/JP2008/073187
(87) International publication number: WO 2009/084477

(57) **Abstract**

An object of the present invention is to provide skin external preparations and cosmetics which contain a branched acyl carnitine and have excellent formulation stability. A skin external preparation of the present invention includes a carnitine derivative represented by the following Formula (1) and/or a carnitine derivative salt represented by the following Formula (2), and an amphoteric surfactant.

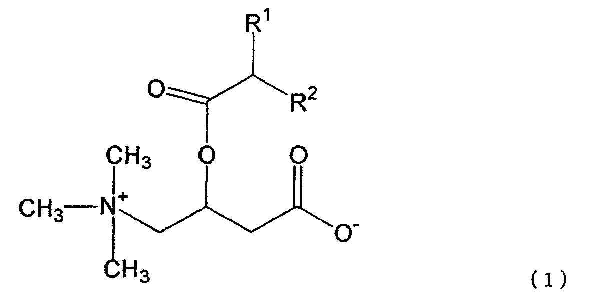

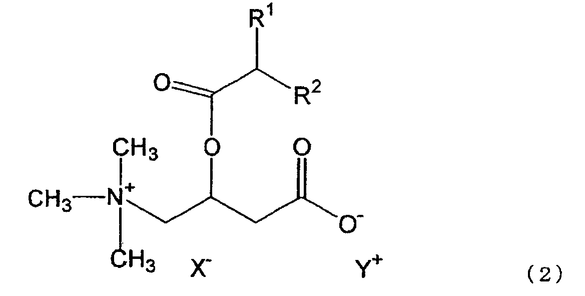

In Formula (1), R¹ and R² are each independently a C1-18 optionally branched, saturated or unsaturated aliphatic hydrocarbon group. In Formula (2), R¹ and R² are the same as in Formula (1), X⁻ is a specific anion and Y⁺ is a specific cation.

## Description

### FIELD OF THE INVENTION

The present invention relates to skin external preparations and cosmetics. In more detail, the invention relates to stable skin external preparations and cosmetics that contain carnitine derivatives and/or salts thereof and specific surfactants and have excellent formulation stability.

### BACKGROUND OF THE INVENTION

Carnitine is well-known to play an important role in human lipid metabolism. In cells, the carnitine is enzymatically bound to the fatty acids from fats and transports the fatty acids into the organelle mitochondria where the fat is burned, serving as a carrier.

On the other hand, excessive lipids lead to obesity as a result of the accumulation of subcutaneous fat, and cause many cosmetic and QOL (quality of life) problems, including cellulite formation, shiny and greasy skin due to excessive sebum, seborrheic dermatitis and consequent hair loss, acnes, body odor, and skin aging due to decreased lipid metabolism.

The lipid metabolism requires that the fatty acids be transported into the mitochondria, and the carnitine is essential to the transportation. Accordingly, the lipid metabolic rate is dependent on the amount of carnitine in the cells. Increasing the carnitine concentration in target tissues for lipid metabolism will stimulate the lipid metabolism and will prevent and eliminate excessive lipids and problems associated therewith.

Many studies have then focused on stimulating the lipid metabolism by percutaneous absorption of carnitine derivatives formulated in skin external preparations. For example, Patent Document 1 discloses a slimming skin external preparation containing palmitoyl-L-carnitine (L-carnitine palmitate). Patent Document 2 proposes a carnitine fatty acid ester having a branched acyl group, and this carnitine derivative has improved stability in formulations.
Patent Document 1: French Patent No. 2694195 specification
Patent Document 2: JP-A-2007-119441

### DISCLOSURE OF THE INVENTION

### PROBLEMS TO BE SOLVED BY THE INVENTION

However, while the branched acyl carnitine is a highly polar, amphoteric substance having a carboxyl group and a quaternary ammonium group in the molecule, the branched acyl group functions as a hydrophobic part. This carnitine derivative is thus hardly dissolved in both aqueous formulations and oil-based formulations and tends to separate from media such as purified water in which the carnitine derivative is dissolved.

The present invention is aimed at solving the problems as described above. It is therefore an object of the invention to provide skin external preparations and cosmetics which comprise branched acyl carnitine and in which the carnitines are unlikely to separate from media.

### MEANS FOR SOLVING THE PROBLEMS

The present inventors studied diligently and have found that branched acyl carnitines in combination with amphoteric surfactants, in particular betaine surfactants, give skin external preparations and cosmetics that are easily formulated to homogeneous formulations with little probability to separate from media. It has been further found that the long-term stability of the formulations is improved by, in addition to the combination, adjusting the pH of the skin external preparations to a specific range. The present invention has been completed based on the findings. In detail, the present invention is concerned with the following [1] to [13].

[1] A skin external preparation comprising a carnitine derivative represented by the following Formula (1) and/or a carnitine derivative salt represented by the following Formula (2), and an amphoteric surfactant;

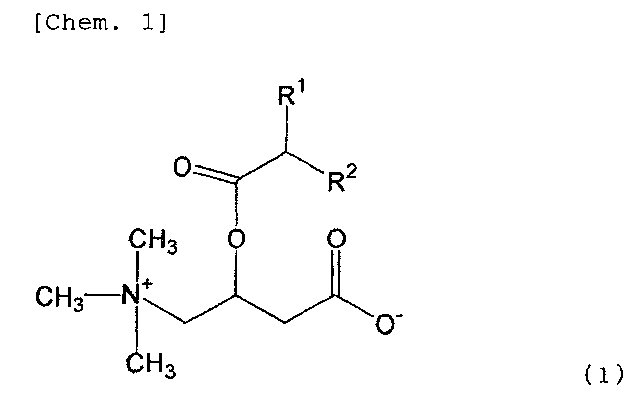

wherein R¹ and R² are each independently a C1-18 optionally branched, saturated or unsaturated aliphatic hydrocarbon group;

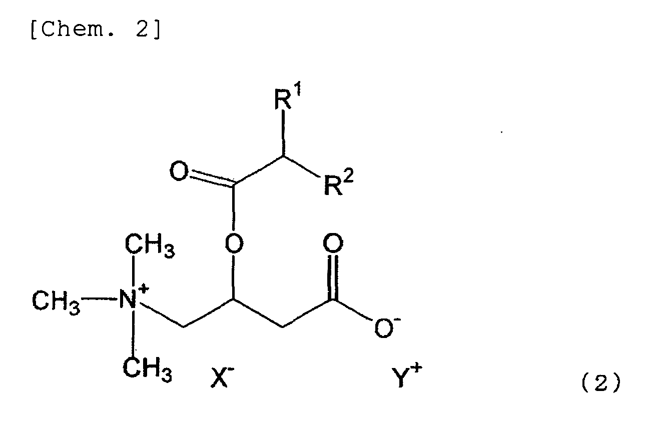

wherein R¹ and R² are the same as R¹ and R² in Formula (1), X⁻ is an inorganic or organic anion providing electrical neutrality with the cation part of the carnitine derivative, and Y⁺ is an inorganic or organic cation providing electrical neutrality with the anion part of the carnitine derivative.

[2] The skin external preparation as described in [1] above, which contains the carnitine derivative and/or the salt thereof at 0.01 to 5% by mass and the amphoteric surfactant in an amount that is 1/10 to 5 times the amount of the carnitine derivative and/or the salt thereof.

[3] The skin external preparation as described in [1] or [2] above, wherein R¹ and R² in Formulae (1) and (2) are each independently a C3-16 optionally branched, saturated or unsaturated aliphatic hydrocarbon group.

[4] The skin external preparation as described in any one of [1] to [3] above, wherein either of R¹ and R² in Formulae (1) and (2) is a C6 linear alkyl group and the other is a C8 linear alkyl group.

[5] The skin external preparation as described in any one of [1] to [4] above, wherein X⁻ in Formula (2) is selected from the group consisting of hydroxide ion, nitrate ion, sulfate ion, carbonate ion, hydrogen carbonate ion, halide ion, formate ion, acetate ion, citrate ion, tartrate ion, oxalate ion, fumarate ion, C3-20 optionally branched, saturated or unsaturated fatty acid anion, carnitine anion, carnitine derivative anion, ascorbate anion, ascorbylphosphate anion and ascorbylphosphate derivative anion.

[6] The skin external preparation as described in any one of [1] to [5] above, wherein Y⁺ in Formula (2) is selected from the group consisting of hydrogen ion, sodium ion, potassium ion, calcium ion, magnesium ion, zinc ion, ammonium ion, carnitine cation and carnitine derivative cation.

[7] The skin external preparation as described in any one of [1] to [6] above, wherein the amphoteric surfactant is a betaine surfactant.

[8] The skin external preparation as described in [7] above, wherein the betaine surfactant is an aminoacetic acid betaine surfactant.

[9] The skin external preparation as described in [7] above, wherein the betaine surfactant is an alkyldimethylaminoacetic acid betaine or a fatty acid amidopropyldimethylaminoacetic acid betaine.

[10] The skin external preparation as described in [7] above, wherein the betaine surfactant is selected from the group consisting of lauryl dimethylaminoacetic acid betaine, cocoalkyl dimethyl glycine, stearyl dimethyl glycine, cocoyl amide propyldimethyl glycine, palm kernel oil amide propyl dimethyl glycine, lauric acid amide betaine propylacetate, propyl betaine ricinoleate amide and stearyl dihydroxyethyl glycine.

[11] The skin external preparation as described in any one of [1] to [10] above, wherein the preparation has a pH in the range of 5.5 to 9.

[12] The skin external preparation as described in any one of [1] to [10] above, wherein the preparation has a pH in the range of 6.0 to 8.0.

[13] The skin external preparation as described in any one of [1] to [12] above, which is used as a cosmetic.

### ADVANTAGEOUS EFFECTS OF THE INVENTION

The skin external preparations and cosmetics of the present invention contain the branched acyl carnitine but are hardly separated from the medium. The formulations thus achieve high homogeneity and stability. In particular, the formulations are easily producible and have long-term storage properties and small changes in appearance, being used in cosmetics in which the touch or appearance greatly influences the commercial values thereof.

Skin external preparations are typically used in small amounts taken out of the container. Maintaining the high homogeneity of the components in the formulations leads to changeless storage and provision without temporal changes of the components in each use of the formulations. The skin external preparations of the present invention have excellent formulation stability and can maintain quality during storage and ensure designed effects of the formulations over a long term after opening till the formulation is finished. The skin external preparations of the present invention can stably provide the effects of the branched acyl carnitine as well as the effects of other components formulated therewith.

### PREFERRED EMBODIMENTS FOR CARRYING OUT THE INVENTION

The present invention will be described in detail below.

A skin external preparation according to the present invention contains a specific carnitine derivative and/or a salt thereof. In detail, the skin external preparation of the present invention contains a specific carnitine derivative or a specific carnitine derivative salt, or contains both a specific carnitine derivative and a salt thereof.

### (Carnitine derivatives)

The carnitine derivatives used in the skin external preparations of the present invention are compounds represented by Formula (1) below:

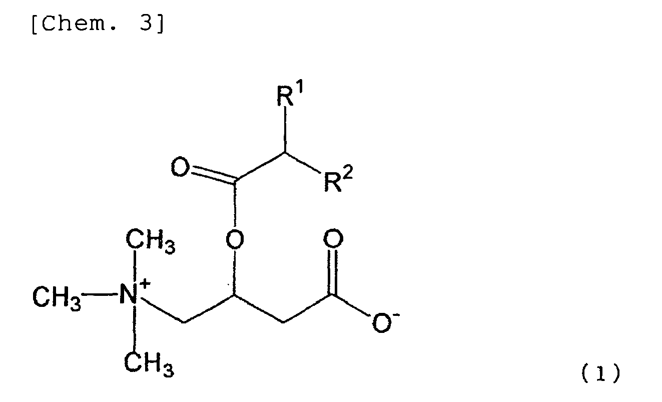

In Formula (1), R¹ and R² are each independently a C1-18 optionally branched, saturated or unsaturated aliphatic hydrocarbon group.

In a preferred embodiment, either of R¹ and R² is a C1-16 optionally branched, saturated or unsaturated aliphatic hydrocarbon group and the other is a C3-16 optionally branched, saturated or unsaturated aliphatic hydrocarbon group. In a more preferred embodiment, R¹ and R² are each independently a C3-16 optionally branched, saturated or unsaturated aliphatic hydrocarbon group. In a still more preferred embodiment, they are each independently a C4-12 optionally branched, saturated or unsaturated aliphatic hydrocarbon group.

The carnitine part in the carnitine derivatives is generally L-form. The carnitine derivatives contain an α-branched acyl group having R¹ and R² When R¹ and R² are different from each other, the α-carbon atom at the branching point is an asymmetric carbon atom, and optical isomers are possible. The carnitine derivatives used in the skin external preparations of the present invention are not particularly limited, and any optical isomers and mixtures thereof may be used.

Examples of the saturated aliphatic hydrocarbon groups include linear or branched alkyl groups such as methyl group, ethyl group, n-propyl group, isopropyl group, n-butyl group, isobutyl group, sec-butyl group, tert-butyl group, n-pentyl group, n-hexyl group, 2-methylpentyl group, 3-methylpentyl group, 4-methylpentyl group, 2-ethylbutyl group, n-heptyl group, 2-methylhexyl group, 3-methylhexyl group, 4-methylhexyl group, 2-ethylpentyl group, 3-ethylpentyl group, n-octyl group, 2-methylheptyl group, 3-methylheptyl group, 4-methylheptyl group, 5-methylheptyl group, 6-methylheptyl group, 2-ethylhexyl group, 3-ethylhexyl group, 4-ethylhexyl group, 2-propylpentyl group, n-nonyl group, n-decyl group, n-undecyl group, n-dodecyl group, n-tridecyl group, n-tetradecyl group, n-pentadecyl group, n-hexadecyl group, n-heptadecyl group, n-octadecyl group and isostearyl group.

Examples of the unsaturated aliphatic hydrocarbon groups include linear or branched alkenyl groups such as 10-undecenyl group, 9-hexadecenyl group, cis-9-octadecenyl, 11-octadecenyl group, cis,cis-9,12-octadecadienyl group, 9,12,15-octadecatrienyl group, 6,9,12-octadecatrienyl group and 9,11,13-octadecatrienyl group.

Preferred combinations of R¹ and R², namely R¹/R², include methyl group/methyl group, methyl group/ethyl group, methyl group/n-propyl group, methyl group/isopropyl group, methyl group/n-butyl group, methyl group/n-pentyl group, methyl group/n-hexyl group, methyl group/n-octyl group, methyl group/n-decyl group, methyl group/n-tetradecyl group, methyl group/n-hexadecyl group, ethyl group/ethyl group, ethyl group/n-propyl group, ethyl group/isopropyl group, ethyl group/n-butyl group, ethyl group/isopropyl group, ethyl group/n-butyl group, ethyl group/n-pentyl group, ethyl group/n-hexyl group, ethyl group/n-octyl group, ethyl group/n-decyl group, ethyl group/n-tetradecyl group, ethyl group/n-hexadecyl group, n-propyl group/n-propyl group, n-propyl group/n-butyl group, n-propyl group/n-pentyl group, n-propyl group/n-hexyl group, n-butyl group/n-hexyl group, n-butyl group/n-octyl group and n-hexyl group/n-octyl group. A particularly preferred combination is n-hexyl group/n-octyl group.

The skin external preparations of the present invention may contain one, or two or more carnitine derivatives specified by the above combination of R¹ and R².

### (Carnitine derivative salts)

The salts of carnitine derivatives used in the skin external preparations of the present invention are compounds represented by Formula (2) below:

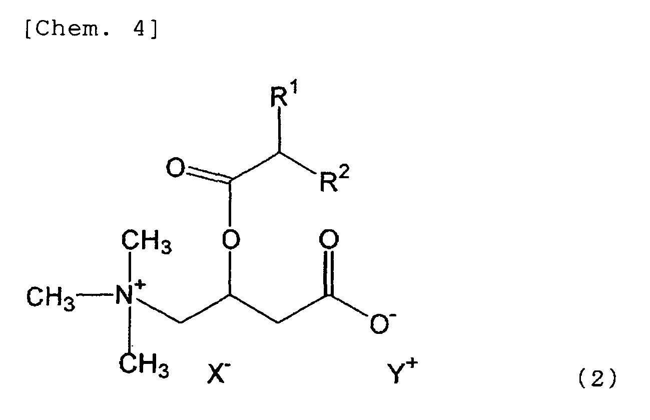

In Formula (2), X⁻ is an inorganic or organic anion providing electrical neutrality with the cation part of the carnitine derivative, and is preferably a pharmaceutically acceptable anion.

Specific examples thereof include inorganic ions such as hydroxide ion, nitrate ion, sulfate ion, carbonate ion, hydrogen carbonate ion and halide ion; and
organic ions such as formate ion, acetate ion, citrate ion, tartrate ion, oxalate ion, fumarate ion, C3-20 optionally branched, saturated or unsaturated fatty acid anion, carnitine anion, carnitine derivative anion, ascorbate anion, ascorbylphosphate anion and ascorbylphosphate derivative anion.

Of these, the hydroxide ion, halide ion, citrate ion, carnitine anion and carnitine derivative anion are preferable from the viewpoint of formulating properties of the skin external preparations, in particular cosmetics.

In Formula (2), Y⁺ is an inorganic or organic cation providing electrical neutrality with the anion part of the carnitine derivative, and is preferably a pharmaceutically acceptable cation.

Specific examples thereof include hydrogen ion, sodium ion, potassium ion, calcium ion, magnesium ion, zinc ion, ammonium ion, carnitine cation and carnitine derivative cation.

Of these, the hydrogen ion, sodium ion, potassium ion, carnitine cation and carnitine derivative cation are preferable from the viewpoint of formulating properties of the skin external preparations, in particular cosmetics.

In Formula (2), R¹ and R² are the same as defined in Formula (1).

The skin external preparations of the present invention may contain one, or two or more carnitine derivative salts specified by the above combination of X⁻, Y⁺, R¹ and R².

### (Amphoteric surfactants)

The skin external preparations of the present invention contain an amphoteric surfactant together with the branched carnitine derivative and/or the salt thereof.

Preferred amphoteric surfactants for use in the present invention include betaine surfactants, in more detail aminoacetic acid betaine surfactants.

Among them, from the viewpoint of formulation stability, alkyldimethylaminoacetic acid betaines and fatty acid amidopropyldimethylaminoacetic acid betaines are particularly preferred.

Specific examples thereof include lauryl dimethylaminoacetic acid betaine, cocoalkyl dimethyl glycine, stearyl dimethyl glycine, cocoyl amide propyldimethyl glycine, palm kernel oil amide propyl dimethyl glycine, lauric acid amide betaine propylacetate, propyl betaine ricinoleate amide and stearyl dihydroxyethyl glycine.

### (Skin external preparations and cosmetics)

The skin external preparations of the present invention contain the aforesaid carnitine derivatives and/or salts thereof, and the amphoteric surfactants. The carnitine derivatives and the salts thereof are effective in improving the lipid metabolism, and may be used for the purposes of improving and preventing skin disorders, aging and obesity associated with excessive lipids or decreased lipid metabolism. The skin external preparations of the present invention may be used as cosmetics, in particular lipid metabolism-improving cosmetics. The skin external preparations of the present invention used as cosmetics will be simply referred to as the cosmetics of the present invention hereinafter.

Examples of the purposes include slimming, cellulite prevention, skin tightening, prevention of shiny and greasy skin and smeared makeup due to excessive sebum, improvement and prevention of seborrheic dermatitis and consequent hair loss, acne prevention, body odor prevention, and anti skin aging, skin activation and skin caring by promoting the conversion of lipids into energy.

The fields of application of the cosmetics according to the present invention are not limited to those described above. The cosmetics of the invention may be used to add the effects of the carnitine derivatives and the salts thereof, to cosmetics having various effects and efficacies.

The skin external preparations of the present invention may contain the carnitine derivatives and/or the salts thereof in amounts corresponding to the desired effects of the carnitine derivatives and/or the salts thereof. From the viewpoint of formulation stability, the content thereof is generally in the range of 0.01 to 10% by mass, preferably 0.01 to 5% by mass, and more preferably 0.05 to 5% by mass based on the total mass of the skin external preparation. The content represents the amount of the carnitine derivative when the carnitine derivative is used alone, or the amount of the carnitine derivative salt when the carnitine derivative salt is used alone, or the total amount of the carnitine derivative and the salt thereof when they are used in combination.

In the skin external preparations of the present invention, the content of the amphoteric surfactant is 1/10 to 5 times, and preferably 1/5 to 2 times the content of the carnitine derivative and/or the salt thereof in terms of % by mass with respect to the total mass of the skin external preparation. This content of the amphoteric surfactants ensures that the skin external preparations have good formulation stability and maintain good conditions and commercial values.

To maintain the formulation stability, the pH of the skin external preparations of the present invention is preferably adjusted in the range of 5.5 to 9.0. In particular, the skin external preparations achieve higher formulation stability and maintain good conditions and commercial values at a pH in the range of 6.0 to 8.0.

The skin external preparations and the cosmetics of the present invention may contain other components commonly used in general skin external preparations and cosmetics in addition to the carnitine derivatives and/or the salts thereof and the amphoteric surfactants, while still achieving the effects of the present invention.

Such components include:

hydrocarbons such as ozokerite, α-olefin oligomers, light isoparaffin, light liquid isoparaffin, squalene, squalane, synthetic squalane, vegetable squalane, ceresin, paraffin, polyethylene powder, polybutene, microcrystalline wax, liquid isoparaffin, liquid paraffin, mineral oil and vaseline;

natural fats and oils, such as natural waxes including jojoba oil, carnauba wax, candelilla wax, rice wax, shellac, lanolin, mink oil wax, spermaceti, sugarcane wax, sperm oil, beeswax and montan wax; avocado oil, almond oil, olive oil, extra virgin olive oil, sesame oil, rice bran oil, rice oil, rice germ oil, corn oil, soybean oil, maize oil, persic oil, palm kernel oil, palm oil, castor oil, grape seed oil, cottonseed oil, coconut oil, hydrogenated coconut oil, tallow, hydrogenated oil, horse oil, mink oil, egg yolk oil, egg yolk fatty oil, rose hip oil, candlenut oil, evening primrose oil, wheat germ oil, peanut oil, tsubaki oil, sasanqua oil, cacao butter, Japan wax, beef bone fat, neatsfoot oil, lard, horse fat, mutton tallow, shea butter, macadamia nut oil and meadowfoam seed oil;

fatty acids such as lauric acid, myristic acid, palmitic acid, stearic acid, behenic acid, oleic acid, isostearic acid, 12-hydroxystearic acid, undecylenic acid and coconut fatty acid;

higher alcohols such as isostearyl alcohol, octyldodecanol, hexyldecanol, cholesterol, phytosterol, lauryl alcohol, myristyl alcohol, cetanol, stearyl alcohol, oleyl alcohol, behenyl alcohol and cetostearyl alcohol;

alkyl glyceryl ethers such as batyl alcohol, chimyl alcohol, selachyl alcohol and isostearyl glyceryl ether;

esters such as isopropyl myristate, butyl myristate, isopropyl palmitate, ethyl stearate, butyl stearate, ethyl oleate, ethyl linoleate, isopropyl linoleate, cetyl caprylate, hexyl laurate, isooctyl myristate, decyl myristate, myristyl myristate, cetyl myristate, octadecyl myristate, cetyl palmitate, stearyl stearate, decyl oleate, oleyl oleate, cetyl ricinoleate, isostearyl laurate, isotridecyl myristate, isocetyl myristate, isostearyl myristate, octyldodecyl myristate, 2-ethylhexyl palmitate, isocetyl palmitate, isostearyl palmitate, 2-ethylhexyl stearate, isocetyl stearate, isodecyl oleate, octyldodecyl oleate, octyldodecyl ricinoleate,
ethyl isostearate, isopropyl isostearate, cetyl 2-ethylhexanoate, cetostearyl 2-ethylhexanoate, stearyl 2-ethylhexanoate, hexyl isostearate, ethylene glycol dioctanoate, ethylene glycol dioleate, propylene glycol dicaprylate, propylene glycol di(caprylate caprate), propylene glycol dicaprate, propylene glycol dioleate, neopentyl glycol dicaprate, neopentyl glycol dioctanoate, glyceryl tricaprylate, glyceryl tri-2-ethylhexanoate, glyceryl tri(caprylate caprate), glyceryl tri(caprylate caprate stearate), glyceryl triundecylate, glyceryl triisopalmitate, glyceryl triisostearate, trimethylolpropane tri-2-ethylhexanoate, trimethylolpropane triisostearate, pentaerythrityl tetra-2-ethylhexanoate, pentaerythrityl tetramyristate,
pentaerythrityl tetraisostearate, diglyceryl tetraisostearate, octyldodecyl neopentanoate, isocetyl octanoate, isostearyl octanoate, 2-ethylhexyl isopelargonate, hexyldecyl dimethyloctanoate, octyldodecyl dimethyloctanoate, 2-ethylhexyl isopalmitate, isocetyl isostearate, isostearyl isostearate, octyldodecyl isostearate, lauryl lactate, myristyl lactate, cetyl lactate, octyldodecyl lactate, triethyl citrate, acetyltriethyl citrate, acetyltributyl citrate, trioctyl citrate, triisocetyl citrate, trioctyldodecyl citrate, diisostearyl malate, 2-ethylhexyl hydroxystearate, di-2-ethylhexyl succinate, diisopropyl adipate, diisobutyl adipate, dioctyl adipate, diheptylundecyl adipate, diethyl sebacate, diisopropyl sebacate, dioctyl sebacate,
cholesteryl stearate, cholesteryl isostearate, cholesteryl hydroxystearate, cholesteryl oleate, dihydrocholesteryl oleate, phytosteryl isostearate, phytosteryl oleate, isocetyl 12-stearoylhydroxystearate, stearyl 12-stearoylhydroxystearate, isostearyl
12-stearoylhydroxystearate, polyoxyethylene (3) polyoxypropylene (1) cetyl ether acetate, polyoxyethylene (3) polyoxypropylene (1) isocetyl ether acetate, isononyl isononanoate, octyl isononanoate, tridecyl isononanoate and isotridecyl isononanoate;

silicone oils such as methyl polysiloxane, methyl phenyl polysiloxane, methyl hydrogen polysiloxane, methyl cyclopolysiloxane, octamethyl cyclotetrasiloxane, decamethyl cyclopentasiloxane, dodecamethyl cyclohexasiloxane, octamethyl trisiloxane, decamethyl tetrasiloxane, tetradecamethyl hexasiloxane, highly polymerized methyl polysiloxane, dimethyl siloxane/methyl (polyoxyethylene) siloxane/methyl (polyoxypropylene) siloxane copolymer, dimethyl siloxane/methyl (polyoxyethylene) siloxane copolymer, dimethyl siloxane/methyl (polyoxypropylene) siloxane copolymer, dimethyl siloxane/methyl cetyloxysiloxane copolymer, dimethyl siloxane/methyl stearoxysiloxane copolymer, polyether-modified silicones, alcohol-modified silicones, alkyl-modified silicones and amino-modified silicones;

polymers such as sodium alginate, carrageenan, agar, furcelleran, guar gum, quince seed, konjac mannan, tamarind gum, tara gum, dextrin, starch, locust bean gum, gum arabic, ghatti gum, karaya gum, tragacanth gum, arabinogalactan, pectin, marmelo, chitosan, starch, curdlan, xanthan gum, gellan gum, cyclodextrin, dextran, pullulan, microcrystalline cellulose, methylcellulose, ethylcellulose, hydroxyethylcellulose, hydroxypropylcellulose, hydroxypropylmethylcellulose, carboxymethylcellulose, carboxy starch, cationized cellulose, starch phosphate, cationized guar gum, carboxymethyl/hydroxypropylated guar gum, hydroxypropylated guar gum, albumin, casein, gelatin, sodium polyacrylate, poly(acrylamide), carboxyvinyl polymers, polyethyleneimine,
highly polymerized polyethylene glycol, polyvinyl alcohol, polyvinylpyrrolidone, polyvinyl ether, polyacrylamide, acrylic acid copolymers, methacrylic acid copolymers, maleic acid copolymers, vinylpyridine copolymers, ethylene/acrylic acid copolymers, vinylpyrrolidone polymers, vinyl alcohol/vinylpyrrolidone copolymers, nitrogen-substituted acrylamide polymers, amino-modified silicones, cationized polymers, dimethylacryl ammonium polymers, acrylate anionic polymers, methacrylate anionic polymers, modified silicones, acrylic acid/alkyl (C₁₀-C₃₀) methacrylate copolymers and polyoxyethylene/polyoxypropylene copolymer;

lower alcohols such as ethanol, isopropyl alcohol, 1-butanol, 2-butanol and benzyl alcohol;

polyhydric alcohols such as ethylene glycol, diethylene glycol, polyethylene glycol, propylene glycol, polypropylene glycol, glycerin, diglycerin, polyglycerin, 1,3-butanediol, triethylene glycol, dipropylene glycol, 3-methyl-1,3-butanediol, 1,2-pentanediol, 1,4-pentanediol, 1,5-pentanediol, 2,4-pentanediol, 2-methyl-2,4-pentanediol, 3-methyl-1,5-pentanediol, 1,2-hexanediol and 1,6-hexanediol;

anionic surfactants such as potassium cocoate, sodium cocoate, triethanolamine cocoate, potassium laurate, sodium laurate, triethanolamine laurate, potassium myristate, sodium myristate, isopropanolamine myristate, potassium palmitate, sodium palmitate, isopropanolamine palmitate, potassium stearate, sodium stearate, triethanolamine stearate, potassium oleate, sodium oleate, sodium castor oil fatty acid, zinc undecylenate, zinc laurate, zinc myristate, magnesium myristate, zinc palmitate, zinc stearate, calcium stearate, magnesium stearate, aluminum stearate, calcium myristate, magnesium myristate, aluminum dimyristate, aluminum isostearate,
polyoxyethylene lauryl ether acetic acid, sodium polyoxyethylene lauryl ether acetate, polyoxyethylene tridecyl ether carboxylic acid, sodium polyoxyethylene tridecyl ether acetate, sodium stearoyl lactate, sodium isostearoyl lactate, sodium N-lauroyl sarcosinate, cocoyl sarcosine, sodium N-cocoyl sarcosinate, triethanolamine N-cocoyl sarcosinate, lauroyl sarcosine, potassium lauroyl sarcosinate, triethanolamine lauroyl sarcosinate, oleoyl sarcosine, sodium myristoyl sarcosinate, sodium stearoyl glutamate, cocoyl glutamic acid, potassium cocoyl glutamate, sodium cocoyl glutamate, triethanolamine cocoyl glutamate, lauroyl acylglutamic acid, potassium lauroyl acylglutamate,
sodium lauroyl acylglutamate, triethanolamine lauroyl acylglutamate, myristoyl acylglutamic acid, potassium myristoyl acylglutamate, sodium myristoyl acylglutamate, stearoyl acylglutamic acid, potassium stearoyl acylglutamate, disodium stearoyl acylglutamate, sodium hydrogenated tallow glutamate, sodium cocoyl glutamate/hydrogenated tallow glutamate, sodium N-methyl N-(1-oxococonut alkyl) β-alanine, lauroyl methyl alanine, sodium lauroyl methyl alanine, triethanolamine lauroyl methyl alanine, sodium myristoyl methyl alanine, sodium lauroyl methyl taurate, potassium N-cocoyl-N-methyl taurate, sodium N-cocoyl-N-methyl taurate, magnesium cocoyl methyl taurate, sodium myristoyl methyl taurate,
sodium palmitoyl methyl taurate, sodium stearoyl methyl taurate, sodium oleoyl methyl taurate, sodium alkane sulfonate, sodium tetradecenesulfonate, sodium dioctyl sulfosuccinate, disodium monolauryl sulfosuccinate, sodium cocoyl ethyl ester sulfonate, sodium lauryl sulfate, triethanolamine lauryl sulfate, sodium cetyl sulfate, triethanolamine alkyl (11, 13, 15) sulfate, sodium alkyl (12, 13) sulfate, triethanolamine alkyl (12, 13) sulfate, ammonium alkyl (12, 14, 16) sulfate, diethanolamine alkyl (12, 13) sulfate, triethanolamine alkyl (12-14) sulfate, triethanolamine alkyl (12-15) sulfate, magnesium ·triethanolamine coco sulfate, ammonium lauryl sulfate, potassium lauryl sulfate, magnesium lauryl sulfate, monoethanolamine lauryl sulfate, diethanolamine lauryl sulfate,
sodium myristyl sulfate, sodium stearyl sulfate, sodium oleyl sulfate, triethanolamine oleyl sulfate, sodium polyoxyethylene lauryl ether sulfate, triethanolamine polyoxyethylene lauryl ether sulfate, sodium polyoxyethylene (1) alkyl (11, 13, 15) ether sulfate, triethanolamine polyoxyethylene (1) alkyl (11, 13, 15) ether sulfate, sodium polyoxyethylene (3) alkyl (11-15) ether sulfate, sodium polyoxyethylene (2) alkyl (12, 13) ether sulfate, sodium polyoxyethylene (3) alkyl (12-14) ether sulfate, sodium polyoxyethylene (3) alkyl (12-15) ether sulfate, sodium polyoxyethylene (2) lauryl ether sulfate, sodium polyoxyethylene (3) myristyl ether sulfate, sodium higher fatty acid alkanolamide sulfate, lauryl monophosphate, sodium lauryl phosphate,
potassium cetyl phosphate, diethanolamine cetyl phosphate, polyoxyethylene oleyl ether phosphate, polyoxyethylene lauryl ether phosphate, sodium polyoxyethylene lauryl ether phosphate, polyoxyethylene cetyl ether phosphate, sodium polyoxyethylene cetyl ether phosphate, polyoxyethylene stearyl ether phosphate, polyoxyethylene oleyl ether phosphate, sodium polyoxyethylene oleyl ether phosphate, polyoxyethylene alkylphenyl ether phosphate, sodium polyoxyethylene alkylphenyl ether phosphate, triethanolamine polyoxyethylene alkylphenyl ether phosphate, polyoxyethylene octyl ether phosphate, polyoxyethylene (10) alkyl (12, 13) ether phosphate, polyoxyethylene alkyl (12-15) ether phosphate, polyoxyethylene alkyl (12-16) ether phosphate, triethanolamine polyoxyethylene lauryl ether phosphate and diethanolamine polyoxyethylene oleyl ether phosphate;

cationic surfactants such as dioctylamine, dimethylstearylamine, trilaurylamine, N-[2-(diethylamino)ethyl] octadecanamide, lauryl trimethyl ammonium chloride, cetyl trimethyl ammonium chloride, cetyl trimethyl ammonium bromide, cetyl trimethyl ammonium saccharinate, stearyl trimethyl ammonium chloride, alkyl (20-22) trimethyl ammonium chloride, lauryl trimethyl ammonium bromide, alkyl (16, 18) trimethyl ammonium chloride, stearyl trimethyl ammonium bromide, stearyl trimethyl ammonium saccharinate, alkyl (28) trimethyl ammonium chloride, di(polyoxyethylene) oleyl methyl ammonium chloride (2EO), dipolyoxyethylene stearyl methyl ammonium chloride, polyoxyethylene (1) polyoxypropylene (25) diethyl methyl ammonium chloride, tri(polyoxyethylene) stearyl ammonium chloride (5EO), distearyl dimethyl ammonium chloride,
dialkyl (12-15) dimethyl ammonium chloride, dialkyl (12-18) dimethyl ammonium chloride, dialkyl (14-18) dimethyl ammonium chloride, dicocoyl dimethyl ammonium chloride, dicetyl dimethyl ammonium chloride, isostearyl lauryl dimethyl ammonium chloride, benzalkonium chloride, myristyl dimethyl benzyl ammonium chloride, lauryl dimethyl (ethylbenzyl) ammonium chloride, stearyl dimethyl benzyl ammonium chloride, lauryl pyridinium chloride, cetyl pyridinium chloride, lauroyl colamino formyl methyl pyridinium chloride, stearoyl colamino formyl methyl pyridinium chloride, alkyl isoquinolium bromide, methyl benzethonium chloride and benzethonium chloride;

nonionic surfactants such as polyoxyethylene (10) alkyl (12, 13) ether, polyoxyethylene lauryl ether, polyoxyethylene cetyl ether, polyoxyethylene stearyl ether, polyoxyethylene oleyl ether, polyoxyethylene (3, 7, 12) alkyl (12-14) ether, polyoxyethylene tridecyl ether, polyoxyethylene myristyl ether, polyoxyethylene-sec-alkyl (14) ether, polyoxyethylene isocetyl ether, polyoxyethylene cetostearyl ether, polyoxyethylene (2, 10, 20) isostearyl ether, polyoxyethylene oleyl cetyl ether, polyoxyethylene (20) arachyl ether, polyoxyethylene octyl dodecyl ether, polyoxyethylene behenyl ether, polyoxyethylene octyl phenyl ether,
polyoxyethylene nonyl phenyl ether, polyoxyethylene dinonyl phenyl ether, polyoxyethylene (1) polyoxypropylene (1, 2, 4, 8) cetyl ether, polyoxyethylene (5) polyoxypropylene (1, 2, 4, 8) cetyl ether, polyoxyethylene (10) polyoxypropylene (1, 2, 4, 8) cetyl ether, polyoxyethylene (20) polyoxypropylene (1, 2, 4, 8) cetyl ether, polyoxyethylene polyoxypropylene lauryl ether, polyoxyethylene (3) polyoxypropylene (34) stearyl ether, polyoxyethylene (4) polyoxypropylene (30) stearyl ether, polyoxyethylene (34) polyoxypropylene (23) stearyl ether, polyoxyethylene polyoxypropylene cetyl ether, polyoxyethylene polyoxypropylene decyl tetradecyl ether,
polyethylene glycol monolaurate, ethylene glycol monostearate, polyethylene glycol monostearate, polyethylene glycol monooleate, ethylene glycol fatty acid ester, self-emulsifying ethylene glycol monostearate, diethylene glycol laurate, polyethylene glycol myristate, polyethylene glycol palmitate, diethylene glycol stearate, self-emulsifying polyethylene glycol (2) monostearate, polyethylene glycol isostearate, ethylene glycol dioctanoate, diethylene glycol dilaurate, polyethylene glycol dilaurate, polyethylene glycol (150) dipalmitate, ethylene glycol distearate, diethylene glycol distearate, polyethylene glycol distearate, ethylene glycol dioleate, polyethylene glycol dioleate,
polyethylene glycol diricinoleate, polyoxyethylene (20) sorbitan monolaurate, polyoxyethylene (20) sorbitan monopalmitate, polyoxyethylene (6) sorbitan monostearate, polyoxyethylene (20) sorbitan monostearate, polyoxyethylene (20) sorbitan tristearate, polyoxyethylene (6) sorbitan monooleate, polyoxyethylene (20) sorbitan monooleate, polyoxyethylene (20) sorbitan trioleate, polyoxyethylene (20) sorbitan monococoate, polyoxyethylene (10-80) sorbitan monolaurate, polyoxyethylene sorbitan tristearate, polyoxyethylene (20) sorbitan isostearate, polyoxyethylene (150) sorbitan tristearate,
polyoxyethylene castor oil, polyoxyethylene hydrogenated castor oil, polyoxyethylene (10) hydrogenated castor oil, polyoxyethylene (20) hydrogenated castor oil, polyoxyethylene (40) hydrogenated castor oil, polyoxyethylene (50) hydrogenated castor oil, polyoxyethylene (60) hydrogenated castor oil, lipophilic glyceryl monostearate, lipophilic glyceryl monooleate, self-emulsifying glyceryl monostearate, glyceryl cocoate, glyceryl laurate, glyceryl myristate, glyceryl isostearate, glyceryl ricinoleate, glyceryl monohydroxystearate, glyceryl oleate, glyceryl linoleate, glyceryl erucate, glyceryl behenate, wheat germ fatty acid glycerides, safflower oil fatty acid glycerin ester, hydrogenated soy glyceride, saturated fatty acid glycerides,
cottonseed oil glyceride, glyceryl monoisostearate monomyristate, glyceryl monotallowate, glyceryl monolanolate, glyceryl sesquioleate, glyceryl distearate, glyceryl diisostearate, glyceryl diarachidate, sorbitan monolaurate, sorbitan monopalmitate, sorbitan monostearate, sorbitan monoisostearate, sorbitan monooleate, sorbitan sesquistearate, sorbitan sesquioleate, sorbitan tristearate, sorbitan trioleate, sorbitan monococoate, sorbitan isostearate, sorbitan sesquiisostearate, sorbitan distearate, diglyceryl isopalmitate, poly (4-10) glyceryl monolaurate, poly (10) glyceryl monomyristate, poly (2-10) glyceryl monostearate, poly (2-10) glyceryl monoisostearate, poly (2-10) glyceryl monooleate, diglyceryl sesquioleate,
poly (2-10) glyceryl diisostearate, poly (6-10) glyceryl distearate, diglyceryl triisostearate, poly (10) glyceryl tristearate, poly (10) glyceryl trioleate, poly (2) glyceryl tetraisostearate, decaglyceryl pentastearate, poly (6-10) glyceryl pentaoleate, poly (10) glyceryl heptastearate, decaglyceryl decastearate, poly (10) glyceryl decaoleate, concentrated poly (6) glyceryl ricinoleate, sucrose fatty acid ester, sucrose cocoate, alkyl glucoside, cocodimethyl amine oxide, lauryl dimethylamine oxide, dihydroxyethyl lauryl dimethylamine oxide, stearyl dimethylamine oxide, oleyl dimethylamine oxide and polyoxyethylene cocoalkyl dimethylamine oxide;

natural surfactants such as saponin, lecithin, soybean phospholipid, hydrogenated soybean phospholipid, soybean lysophospholipid, hydrogenated soybean lysophospholipid, egg yolk lecithin, hydrogenated egg yolk lysophosphatidylcholine, phosphatidylcholine, phosphatidylethanolamine, phosphatidylserine, sphingophospholipid, sphingomyelin, ganglioside, bile acid, cholic acid, deoxycholic acid, sodium cholate, sodium deoxycholate, spiculisporic acid, rhamnolipid, trehalose lipid, sophorolipid and mannosylerythritol lipid;

ultraviolet absorbers, including para-aminobenzoic acid derivatives such as para-aminobenzoic acid, ethyl para-aminobenzoate, glyceryl para-aminobenzoate, amyl para-dimethylaminobenzoate and 2-ethylhexyl para-dimethylaminobenzoate, cinnamic acid derivatives such as benzyl cinnamate, glyceryl 2-ethylhexanoate di-para-methoxy cinnamate, 2,4-diisopropyl methyl cinnamate, 2,4-diisopropyl ethyl cinnamate, potassium para-methoxycinnamate, sodium para-methoxycinnamate, isopropyl para-methoxycinnamate, 2-ethylhexyl para-methoxycinnamate, 2-ethoxyethyl paramethoxycinnamate and ethyl para-ethoxycinnamate, urocanic acid derivatives such as urocanic acid and ethyl urocanate,
benzophenone derivatives such as 2,4-dihydroxybenzophenone, 2,2',4,4'-tetrahydroxybenzophenone, sodium 2-hydroxy-4-methoxy-5-sulfobenzophenone, 2-hydroxy-4-methoxybenzophenone-5-sulfonic acid, 2-hydroxy-4-methoxybenzophenone, 2,2'-dihydroxy-4,4'-dimethoxybenzophenone and sodium 2,2'-dihydroxy-4,4'-dimethoxy-5-sulfobenzophenone, salicylic acid derivatives such as ethylene glycol salicylate, 2-ethylhexyl salicylate, phenyl salicylate, benzyl salicylate, p-tert-butylphenyl salicylate, homomenthyl salicylate and 3,3,5-trimethylcyclohexyl salicylate, 2-(2'-hydroxy-5'-methoxyphenyl)benzotriazole and 4-tert-butyl-4'-methoxybenzoylmethane;

powders and color materials such as kaolin, silicic anhydride, aluminum magnesium silicate, sericite, talc, boron nitride, mica, montmorillonite, ramie cellulose powder, wheat starch, silk powder, cornstarch, nitro dye, azo dye, nitroso dye, triphenylmethane dye, xanthene dye, quinoline dye, anthraquinone dye, indigo dye, pyrene dye, phthalocyanine dye,
natural dyes including flavonoid, quinone, porphyrin, water-soluble annatto, cuttlefish ink powder, caramel, guaiazulene, gardenia blue, gardenia yellow, cochineal, shikonin, sodium copper chlorophyllin, paprika dye, safflower red, safflower yellow, laccaic acid and riboflavin butyrate, carbon black, yellow iron oxide, black iron oxide, red iron oxide, iron blue, ultramarine blue, zinc oxide, chromium oxide, titanium oxide, black titanium oxide, zirconium oxide, chromium hydroxide, alumina, magnesium oxide, barium sulfate, aluminum hydroxide, calcium carbonate, lithium cobalt titanate, manganese violet and pearl pigment;

plant extracts such as angelica extract, gambir extract, avocado extract, hydrangea extract, gynostemma pentaphyllum extract, althea extract, arnica extract, oil-soluble arnica extract, almond extract, aloe extract, styrax resin extract, ginkgo extract, nettle extract, orris extract, fennel extract, turmeric extract, rose fruit extract, echinacea leaf extract, scutellaria root extract, phellodendron bark extract, Japanese coptis extract, barley extract, okura extract, hypericum extract, oil-soluble hypericum extract, white nettle extract, oil-soluble white nettle extract, restharrow extract, watercress extract, orange flower water, persimmon tannin, pueraria root extract, Japanese valerian extract, cattail extract, chamomile extract, oil-soluble chamomile extract, chamomile distillate, oat extract, carrot extract, oil-soluble carrot extract, carrot oil, artemisia capillaris extract, glycyrrhiza extract, glycyrrhiza extracted powder, glycyrrhiza flavonoid, cantharides tincture, raspberry extract, kiwi extract, cinchona extract,
cucumber extract, apricot kernel extract, quince seed extract, gardenia extract, sasa albo-marginata extract, sophora root extract, walnut shell extract, clematis extract, black sugar extract, chlorella extract, mulberry root extract, cinnamon bark extract, gentian extract, geranium herb extract, black tea extract, nuphar extract, burdock root extract, oil-soluble burdock root extract, wheat germ extract, hydrolyzed wheat powder, rice bran extract, fermented rice bran extract, comfrey extract, asiasarum root extract, saffron extract, saponaria extract, oil-soluble salvia extract, crataegus fruit extract, zanthoxylum fruit extract, shiitake mushroom extract, shiitake mushroom extracted powder, rehmannia root extract, lithospermum root extract, oil-soluble lithospermum root extract, perilla herb extract, linden extract, oil-soluble linden extract, filipendula extract, peony root extract, Job's tears extract, ginger extract, oil-soluble ginger extract, ginger tincture, calamus rhizome extract, birch extract, oil-soluble birch extract, birch sap, honeysuckle extract, horsetail extract, oil-soluble horsetail extract,
scordinine, stevia extract, ivy extract, crataegus extract, sambucus extract, juniper extract, yarrow extract, oil-soluble yarrow extract, peppermint extract, sage extract, oil-soluble sage extract, sage water, mallow extract, celery extract, cnidium rhizome extract, cnidium rhizome water, swertia herb extract, soy extract, jujube extract, thyme extract, green tea extract, tea leaf dry distilled solution, tea seed extract, clove extract, citrus unshiu peel extract, camellia extract, centella extract, oil-soluble walnut extract, duke extract, terminalia extract, Japanese angelica root extract, oil-soluble Japanese angelica root extract, Japanese angelica root water, calendula extract, oil-soluble calendula extract, soy milk powder, peach seed extract, bitter orange peel extract, houttuynia extract, tomato extract, tormentilla extract, natto extract, ginseng extract, oil-soluble ginseng extract, garlic extract, wild rose extract,
oil-soluble wild rose extract, malt extract, malt root extract, ophiopogon tuber extract, parsley extract, barley leaf juice concentrate, peppermint distillate, witch hazel distillate, witch hazel extract, rose extract, pellitory extract, isodonis extract, loquat leaf extract, oil-soluble loquat leaf extract, coltsfoot extract, hoelen extract, butcher broom extract, butcher broom extracted powder, grape extract, grape leaf extract, grape water, hayflower extract, sponge gourd extract, sponge gourd solution, safflower extract, oil-soluble linden extract, linden water, paeonia extract, hop extract, oil-soluble hop extract, pine extract, milk thistle extract, horse chestnut extract, oil-soluble horse chestnut extract, mukurossi peel extract, balm mint extract, sweet clover extract, peach leaf extract, oil-soluble peach leaf extract, bean sprouts extract, cornflower extract, cornflower distillate, eucalyptus extract, saxifrage extract, lily extract, coix extract, oil-soluble coix extract, mugwort extract, Japanese mugwort water, lavender extract, lavender water, apple extract, ganoderma extract, lettuce extract, Chinese milk vetch extract, rose water, rosemary extract, oil-soluble rosemary extract, Romanchamomile extract and burnet extract;

amino acids and peptides such as glycine, valine, leucine, isoleucine, serine, threonine, phenylalanine, tyrosine, tryptophan, cystine, cysteine, methionine, hydroxyproline, aspartic acid, asparagine, glutamic acid, glutamine, histidine, γ-aminobutyric acid, DL-pyrrolidonecarboxylic acid, ε-aminocaproic acid, hydrolyzed elastin, water-soluble elastin, hydrolyzed collagen, water-soluble collagen, casein, glutathione, wheat peptide and soybean peptide;

vitamins and vitamin-like substances, including vitamin A such as retinol, retinal, retinoic acid, retinol acetate and retinol palmitate, carotenoids such as α-carotene, β-carotene, γ-carotene, δ-carotene, lycopene, zeaxanthin, cryptoxanthin, echinenone and astaxanthin, vitamin B1 such as thiamines, vitamin B2 such as riboflavin, vitamin B6 such as pyridoxine, pyridoxal and pyridoxamine, vitamin B12 such as cyanocobalamin,
vitamin C such as folic acids, nicotinic acid, nicotinamide, pantothenic acids, biotins, L-ascorbic acid, sodium L-ascorbate, L-ascorbyl stearate, L-ascorbyl palmitate, L-ascorbyl dipalmitate, L-ascorbyl tetraisopalmitate, disodium L-ascorbic acid sulfate, L-ascorbyl magnesium, sodium L-ascorbyl phosphate, ascorbic acid-2-phosphate and L-ascorbic acid-2-glucoside, vitamin D such as ergocalciferol and cholecalciferol, vitamin E such as d-α-tocopherol, DL-α-tocopherol, dl-α-tocopherolacetate, dl-α-tocopherol succinate, β-tocopherol, γ-tocopherol and d-δ-tocopherol, ubiquinones, vitamin K, carnitine, ferulic acid, γ-oryzanol, α-lipoic acid and orotic acid;

preservative agents such as benzoic acid, sodium benzoate, undecylenic acid, salicylic acid, sorbic acid, potassium sorbate, dehydroacetic acid, sodium dehydroacetate, isobutyl paraoxybenzoate, isopropyl paraoxybenzoate, ethyl paraoxybenzoate, butyl paraoxybenzoate, propyl paraoxybenzoate, benzyl paraoxybenzoate, methyl paraoxybenzoate, sodium methyl paraoxybenzoate, phenoxyethanol, photosensitizing dye No. 101, photosensitizing dye No. 201 and photosensitizing dye No. 401;

antioxidants such as butylhydroxyanisol, butylhydroxytoluene, propyl gallate, erythorbic acid, sodium erythorbate, para-hydroxyanisol and octyl gallate;

sequestering agents, including metal ionic compounds such as trisodium hydroxyethyl ethylenediamine triacetate, edetic acid, disodium edetate, trisodium edetate, tetrasodium edetate, sodium citrate, gluconic acid, phytic acid, sodium polyphosphate and sodium metaphosphate;

moisturizers such as hyaluronic acid, sodium hyaluronate, sodium chondroitin sulfate, sodium lactate, sodium pyrrolidone carboxylate, betaine, lactic acid bacteria culture, yeast extract and ceramide;

anti-inflammatory agents such as glycyrrhizinic acid, trisodium glycyrrhizinate, dipotassium glycyrrhizinate, monoammonium glycyrrhizinate, β-glycyrrhetinic acid, glyceryl glycyrrhetinate, stearyl glycyrrhetinate, lysozyme chloride, hydrocortisone and allantoin;

pH adjusters such as sodium hydroxide, potassium hydroxide and triethanolamine;

salts such as sodium chloride, potassium chloride, magnesium chloride and sodium sulfate;

α-hydroxy acids such as citric acid, glycolic acid, tartaric acid and lactic acid;

whitening agents such as arbutin, α-arbutin and placental extract;

essential oils such as angelica oil, ylang ylang oil, elemi oil, chamomile oil, Romanchamomile oil, cardamom oil, calamus oil, galbanum oil, camphor oil, carrot seed oil, clary sage oil, clove oil, cinnamon oil, coriander oil, cypress oil, sandalwood oil, cedarwood oil, citronella oil, cinnamon leaf oil, jasmine absolute, juniperberry oil, ginger extract, spearmint oil, sage oil, cedar oil, geranium oil, thyme oil, tea tree oil, nutmeg oil, niaouli oil, neroli oil, pine oil, basil oil, mentha oil, patchouli oil, palmarosa oil, fennel oil, petitgrain oil, black pepper oil, frankincense oil, vetivert oil, peppermint oil, bergamot oil, benzoin oil, aniba rosaeodora oil, marjoram oil, myrrh oil, balm mint oil, eucalyptus oil, ravensara oil, lavandin oil, lavender oil, linden oil, rose oil, rosewood oil, rosemary oil and lovage oil;

terpenes such as pinene, terpinene, terpinolene, myrcene and longifolene;
perfumes and water.

The formulation types and forms of the skin external preparations and the cosmetics of the present invention are not particularly limited as long as they are used directly on skin. In a preferred embodiment, the skin external preparations and cosmetics are applied to the skin in the vicinity of where subcutaneous fat metabolism is desired.

In the broad sense, the skin external preparations and the cosmetics include any formulations that are used directly on skin, such as skin milks, skin creams, foundation creams, massage creams, cleansing creams, shaving creams, cleansing foams, skin toners, lotions, packs, lipsticks, rouges, eye shadows, manicures, soaps, body shampoos, hand soaps, shampoos, rinses, hair tonics, treatment conditioners, hair creams, hair sprays, hair growth tonics, baldness remedies, hairdyes, styling spritz, depilatories, antidandruff agents, toothpastes, denture adhesives, mouthwashes, permanent waving agents, curling agents, styling agents, ointments, adhesive skin patches, taping agents, bath agents, antiperspirants and sunscreen agents. The skin external preparations and the cosmetics may be used regardless of user' s gender and age, and may be used for animal skin as well as human skin.

The skin external preparations and the cosmetics according to the present invention may be in any forms including solids, liquids, semisolids, powders, granules, tablets, gels, foams and sprays.

Although not particularly limited, the formulation types and forms containing aqueous media are preferably selected because the present invention is particularly effective in improving the poor stability of the conventional carnitine derivatives and salts thereof in aqueous formulations. In this case, water may be usually used in an amount of 0.01 to 99.99% by mass of the skin external preparations or cosmetics of the present invention.

The cosmetics according to the present invention may further contain existing cosmetic ingredients in addition to the above-described ingredients. For example, all of the cosmetic ingredients listed in The Japanese Standards of Cosmetic Ingredients 2nd edition (edited by Pharmaceutical and Medical Device Regulatory Science Society of Japan and published by Yakuji Nippo, Ltd. (1984)), The Japanese Cosmetic Ingredients Codex (edited by Ministry of Health and Welfare, Pharmaceutical Affairs Bureau, Examination Division and published by Yakuji Nippo, Ltd. (1993)), Supplement to The Japanese Cosmetic Ingredients Codex (edited by Ministry of Health and Welfare, Pharmaceutical Affairs Bureau, Examination Division and published by Yakuji Nippo, Ltd. (1993)), The Comprehensive Licensing Standards of Cosmetics by Category (edited by Ministry of Health and Welfare, Pharmaceutical Affairs Bureau, Examination Division and published by Yakuji Nippo, Ltd. (1993)), The Japanese Cosmetic Ingredients Codex by Category (edited by Ministry of Health and Welfare, Pharmaceutical Affairs Bureau, Examination Division and published by Yakuji Nippo, Ltd. (1997)), Keshouhin Genryou Jiten (Dictionary of Cosmetic Ingredients) (Nikko Chemicals., Co. Ltd. (1991)), and Atarashii Keshouhin Kinou Sozai 300 (300 Latest Cosmetic Functional Materials) (CMC Publishing Co., Ltd. (2002)) can be used while still achieving the advantageous effects of the present invention.

The skin external preparations and the cosmetics of the present invention may be produced by common methods depending on the formulation types, for example by dissolving, mixing or dispersing the aforesaid ingredients in predetermined amounts.

### EXAMPLES

The present invention will be described in greater detail based on examples below. However, it should be construed that the invention is not limited to such examples. In Examples, % is percent by mass unless otherwise specified.

### [Synthetic Example 1]

### Synthesis of L-carnitine 2-hexyldecanoate hydrochloride

In an ice bath, 45.6 g (0.283 mol) of L-carnitine was dissolved in 150 ml of trifluoroacetic acid. To the resultant solution, 116.6 g (0.425 mol) of 2-hexyldecanoic acid chloride was added dropwise over a period of 10 minutes, followed by stirring at 80°C for 4 hours. The solvent was distilled away under reduced pressure. The resultant dark brown oily substance weighing 264.6 g was washed with 200 ml of n-hexane three times, and 200 ml of a black oily substance was obtained. A 3.0 g portion of the oily substance was subjected to extraction with 20 ml of ethanol, 30 ml of n-butanol and 60 ml of water. The organic phase obtained was washed with 60 ml of water, and with a mixture of 20 ml of ethanol and 60 ml of water. The obtained organic phase was further washed with 60 ml of water, collected and dried. Evaporating the solvent resulted in a yield of 2.0 g of L-carnitine 2-hexyldecanoate hydrochloride.

The structure of the target compound was identified by NMR, liquid chromatography-mass spectrometry (LC/MS) and elemental analysis as follows.

### (NMR)

¹H-NMR (CDCl₃): 0.90 ppm (6H, t, 7.0 Hz), 1.29-1.62 ppm (24H, m), 2. 41 ppm (1H, m), 2. 76 ppm (2H, d, 6. 0 Hz), 3.21 ppm (9H, s), 3.75 ppm (1H, d, 14.6 Hz), 3.92 ppm (1H, dd, 8.2, 14.6 Hz), 5.60-5.64 ppm (1H, m)
NMR apparatus: Burker Advance 500
Sample concentration: 40 mg sample/422 mg deuterated chloroform
Temperature: room temperature

### (Liquid chromatography-mass spectrometry (LC/MS))

MS (ESI) m/z: 400.5 [M⁺] (LC/MS)
Liquid chromatograph (LC): Agilent 1100 series
Column: Shodex silica C8-5B
Column temperature: 40°C
Eluting solution: 20 mM aqueous ammonium acetate solution/acetonitrile = 30/70
Flow rate: 1.0 ml/min
Sample concentration and injection amount: 10 µl x 5 mg/ml eluting solution
Detection: photodiode array UV 200-700 nm
Mass spectrometer (MS): Thermoquest LCQ Advantage
Ionizing method: ESI (electrospray ionization)
Scan range: m/z 50-1000 (alternate positive and negative charges)
MS/MS collision energy: 40%.

### (Elemental analysis)

Elemental analysis: C: 62.8%, H: 10.60, N: 3.5%, O: 14.9%, C1: 8.2%
C, H, N and O: Organic elemental analyzer CHNS-932 and oxygen analysis option VTF-900 (manufactured by LECO)
References: sym-diphenylthiourea (for analysis of C, H and N), and p-nitroaniline (for analysis of O)
C1: ion chromatography (Exactly 1 mg of the sample was weighed and mixed with an eluting solution (1.8 mM Na₂CO₃ + 1. 7 mM NaHCO₃ to a constant volume of 100 ml, and analyzed with an anion chromatograph (DIONEX DX-500) to determine C1 in the sample.)
Column: Shodex SI-90 4E
Flow rate: 1.0 ml/min
Injection amount: 25 µl
Detector: Electrical conductivity detector
Suppressor: ASRS-I.

### [Synthetic Example 2]

### Synthesis of L-carnitine 2-methylpalmitate hydrochloride

In an ice bath, 45.6 g (0.283 mol) of L-carnitine was dissolved in 150 ml of trifluoroacetic acid. To the resultant solution, 122. 4 g (0.425 mol) of 2-methylpalmitic acid chloride was added dropwise over a period of 10 minutes, followed by stirring at 80°C for 4 hours. The solvent was distilled away under reduced pressure. The resultant dark brown oily substance weighing 278.0 g was washed with 200 ml of n-hexane three times, and 200 ml of a black oily substance was obtained. A 3. 0 g portion of the oily substance was subjected to extraction with 20 ml of ethanol, 30 ml of n-butanol and 60 ml of water. The organic phase obtained was washed with 60 ml of water, and with a mixture of 20 ml of ethanol and 60 ml of water. The obtained organic phase was further washed with 60 ml of water, collected and dried. Evaporating the solvent resulted in a yield of 2.2 g of L-carnitine 2-methylpalmitate hydrochloride.

The structure of the target compound was identified by NMR, liquid chromatography-mass spectrometry (LC/MS) and elemental analysis as described in Synthetic Example 1, the results being shown below.

### (NMR)

¹H-NMR (CDCl₃): 2.50 ppm (1H, m), 2.75 ppm (1H, dd, 6.2, 1.5 Hz), 2.80 ppm (1H, m), 3.73 ppm (1H, d, 14.4 Hz), 3.91 ppm (1H, dd, 14.4, 8.5 Hz), 5.61-5.65 ppm (1H, m).

### <Liquid chromatography-mass spectrometry (LC/MS)>

MS (ESI) m/z: 414.5 [M⁺]

### (Elemental analysis)

C: 64.3%, H: 10.3%, N: 3.2%, O: 14.2%, C1: 8.0%.

### [Synthetic Example 3]

### Synthesis of L-carnitine 2-butyloctanoate hydrochloride

In an ice bath, 45.6 g (0.283 mol) of L-carnitine was dissolved in 150 ml of trifluoroacetic acid. To the resultant solution, 92.7 g (0.425 mol) of 2-butyloctanoic acid chloride was added dropwise over a period of 10 minutes, followed by stirring at 80°C for 4 hours. The solvent was distilled away under reduced pressure. The resultant dark brown oily substance weighing 250.0 g was washed with 200 ml of n-hexane three times, and 200 ml of a black oily substance was obtained. A 3.0 g portion of the oily substance was subjected to extraction with 20 ml of ethanol, 30 ml of n-butanol and 60 ml of water. The organic phase obtained was washed with 60 ml of water, and with a mixture of 20 ml of ethanol and 60 ml of water. The obtained organic phase was further washed with 60 ml of water, collected and dried. Evaporating the solvent resulted in a yield of 1.6 g of L-carnitine 2-butyloctanoate hydrochloride.

The structure of the target compound was identified by NMR, liquid chromatography-mass spectrometry (LC/MS) and elemental analysis as described in Synthetic Example 1, the results being shown below.

### (NMR)

¹H-NMR (CDCl₃): 2.76 ppm (1H, d, 6.0 Hz), 2.80 ppm (1H, m), 3.76 ppm (1H, d, 14.4 Hz), 3.94 ppm (1H, dd, 14.4, 8.2 Hz), 5.60-5.64 ppm (1H, m).

### <Liquid chromatography-mass spectrometry (LC/MS)>

MS (ESI) m/z: 344.5 [M⁺]

### (Elemental analysis)

C: 60.1%, H: 9.5%, N: 3.5%, O: 16.7%, Cl: 10.2%.

### [Formulation Examples 1 to 5]

Cosmetics were prepared according to the formulations shown in Table 1 below.

Ingredients were blended according to the compositions described in the columns for cosmetics 1 to 5 in Table 1 below, and were mixed together with a homomixer at 60°C. The mixtures were continuously stirred while being ice-cooled to approximately 30°C, and were thereafter naturally cooled to room temperature to give cosmetics 1 to 5 that contained the L-carnitine 2-hexyldecanoate hydrochloride prepared in Synthetic Example 1.

For the cosmetic 4, a nonionic, hydrogenated castor oil surfactant was used instead of betaine surfactants. The cosmetic 5 was a reference cosmetic containing no betaine surfactants.

These cosmetics were placed in glass vials, and the conditions of the formulations were visually observed immediately after the preparation, after 24 hours of standing, and after 1 month of standing. The results are set forth in Table 1.

### [Table 1]

**Table 1**

| Ingredients | Amounts (%) | | | | |
|---|---|---|---|---|---|
| | Cosmetic 1 | Cosmetic 2 | Cosmetic 3 | Cosmetic 4 | Cosmetic 5 |
| Glycerin | 4.0 | 4.0 | 4.0 | 4.0 | 4.0 |
| 1,3-Butylene glycol | 4.0 | 4.0 | 4.0 | 4.0 | 4.0 |
| 10% (w/v) Potassium hydroxide | 0.5 | 0.5 | 0.5 | 0.5 | 0.5 |
| Methylparaben | 0.1 | 0.1 | 0.1 | 0.1 | 0.1 |
| L-carnitine 2-hexyldecanoate hydrochloride | 0.5 | 0.5 | 0.5 | 0.5 | 0.5 |
| Cocoyl amide propyldimethyl glycine | 0.1 | | | | |
| Lauryl dimethylaminoacetic acid betaine | | 0.1 | | | |
| Lauric acid amide betaine propylacetate | | | 0.1 | | |
| Polyoxyethylene hydrogenated castor oil 50 | | | | 0.1 | |
| Purified water | Balance | Balance | Balance | Balance | Balance |

| Conditions | | | | | |
|---|---|---|---|---|---|
| Immediately after preparation | Homogeneous Transparent | Homogeneous Transparent | Homogeneous Transparent | White turbid | White turbid |
| 24 Hours after preparation | Homogeneous Transparent | Homogeneous Transparent | Homogeneous Transparent | Separated | Separated |
| 1 Month after preparation | Homogeneous Transparent | Homogeneous Transparent | Homogeneous Transparent | (Not observed) | (Not observed) |

| | | | | | |
|---|---|---|---|---|---|
| * The values are weight percentages relative to the total weight at 100. | | | | | |

### [Formulation Examples 6 to 10]

Cosmetics were prepared according to the formulations shown in Table 2 below.

Ingredients were blended according to the compositions described in the columns for cosmetics 6 to 10 in Table 2 below, and were mixed together with a homomixer at 60°C. The mixtures were continuously stirred while being ice-cooled to approximately 30°C, and were thereafter naturally cooled to room temperature to give cosmetics 6 to 10 that contained the L-carnitine 2-methylpalmitate hydrochloride prepared in Synthetic Example 2.

For the cosmetic 9, a nonionic, hydrogenated castor oil surfactant was used instead of betaine surfactants. The cosmetic 10 was a reference cosmetic containing no betaine surfactants.

These cosmetics were placed in glass vials, and the conditions of the formulations were visually observed immediately after the preparation, after 24 hours of standing, and after 1 month of standing. The results are set forth in Table 2.

### [Table 2]

**Table 2**

| Ingredients | Amounts (%) | | | | |
|---|---|---|---|---|---|
| | Cosmetic 6 | Cosmetic 7 | Cosmetic 8 | Cosmetic 9 | Cosmetic 10 |
| Glycerin | 4.0 | 4.0 | 4.0 | 4.0 | 4.0 |
| 1,3-Butylene glycol | 4.0 | 4.0 | 4.0 | 4.0 | 4.0 |
| 1,2-Hexanediol | 5.0 | 5.0 | 5.0 | 5.0 | 5.0 |
| 10% (w/v) Potassium hydroxide | 0.5 | 0.5 | 0.5 | 0.5 | 0.5 |
| Methylparaben | 0.1 | 0.1 | 0.1 | 0.1 | 0.1 |
| L-carnitine 2-methylpalmitate hydrochloride | 0.5 | 0.5 | 0.5 | 0.5 | 0.5 |
| Cocoyl amide propyldimethyl glycine | 2.5 | | | | |
| Lauryl dimethylaminoacetic acid betaine | | 2.5 | | | |
| Lauric acid amide betaine propylacetate | | | 2.5 | | |
| Polyoxyethylene hydrogenated castor oil 50 | | | | 2.5 | |
| Purified water | Balance | Balance | Balance | Balance | Balance |

| Conditions | | | | | |
|---|---|---|---|---|---|
| Immediately after preparation | Homogeneous Transparent | Homogeneous Transparent | Homogeneous Transparent | White turbid | White turbid |
| 24 Hours after preparation | Homogeneous Transparent | Homogeneous Transparent | Homogeneous Transparent | Precipitated | Precipitated |
| 1 Month after preparation | Homogeneous Transparent | Homogeneous Transparent | Homogeneous Transparent | (Not observed) | (Not observed) |

| | | | | | |
|---|---|---|---|---|---|
| * The values are weight percentages relative to the total weight at 100. | | | | | |

### [Formulation Examples 11 to 15]

Cosmetics were prepared according to the formulations shown in Table 3 below.

Ingredients were blended according to the compositions described in the columns for cosmetics 11 to 15 in Table 3 below, and were mixed together with a homomixer at 60°C. The mixtures were continuously stirred while being ice-cooled to approximately 30°C, and were thereafter naturally cooled to room temperature to give cosmetics 11 to 15 that contained the L-carnitine 2-butyloctanoate hydrochloride prepared in Synthetic Example 3.

For the cosmetic 14, a nonionic, hydrogenated castor oil surfactant was used instead of betaine surfactants. The cosmetic 15 was a reference cosmetic containing no betaine surfactants.

These cosmetics were placed in glass vials, and the conditions of the formulations were visually observed immediately after the preparation, after 24 hours of standing, and after 1 month of standing. The results are set forth in Table 3.

### [Table 3]

**Table 3**

| Ingredients | Amounts (%) | | | | |
|---|---|---|---|---|---|
| | Cosmetic 11 | Cosmetic 12 | Cosmetic 13 | Cosmetic 14 | Cosmetic 15 |
| Glycerin | 4.0 | 4.0 | 4.0 | 4.0 | 4.0 |
| 1,3-Butylene glycol | 4.0 | 4.0 | 4.0 | 4.0 | 4.0 |
| 10% (w/v) Potassium hydroxide | 0.6 | 0.6 | 0.6 | 0.6 | 0.6 |
| Methylparaben | 0.1 | 0.1 | 0.1 | 0.1 | 0.1 |
| L-carnitine 2-butyloctanoate hydrochloride | 0.5 | 0.5 | 0.5 | 0.5 | 0.5 |
| Cocoyl amide propyldimethyl glycine | 1.5 | | | | |
| Lauryl dimethylaminoacetic acid betaine | | 1.5 | | | |
| Lauric acid amide betaine propylacetate | | | 1.5 | | |
| Polyoxyethylene hydrogenated castor oil 50 | | | | 1.5 | |
| Purified water | Balance | Balance | Balance | Balance | Balance |

| Conditions | | | | | |
|---|---|---|---|---|---|
| Immediately after preparation | Homogeneous Slightly turbid | Homogeneous Slightly turbid | Homogeneous Slightly turbid | White turbid | White turbid |
| 24 Hours after preparation | Homogeneous Slightly turbid | Homogeneous Slightly turbid | Homogeneous Slightly turbid | Phase separation | Phase separation |
| 1 Month after preparation | Homogeneous Slightly turbid | Homogeneous Slightly turbid | Homogeneous Slightly turbid | (Not observed) | (Not observed) |

| | | | | | |
|---|---|---|---|---|---|
| * The values are weight percentages relative to the total weight at 100. | | | | | |

### [Formulation Examples 16 to 18]

Cosmetics were prepared according to the formulations shown in Table 4 below.

Ingredients were blended according to the compositions described in the columns for cosmetics 16 to 18 in Table 4 below, and were mixed together with a homomixer at 60°C. The mixtures were continuously stirred while being ice-cooled to approximately 30°C, and were thereafter naturally cooled to room temperature to give cosmetics 16 to 18 that contained the L-carnitine 2-hexyldecanoate hydrochloride prepared in Synthetic Example 1.

These cosmetics were placed in glass vials, and the conditions of the formulations were visually observed immediately after the preparation, after 24 hours of standing, and after 1 month of standing. The results are set forth in Table 4.

### [Table 4]

**Table 4**

| Ingredients | Amounts (%) | | |
|---|---|---|---|
| | Cosmetic 16 | Cosmetic 17 | Cosmetic 18 |
| Glycerin | 4.0 | 4.0 | 4.0 |
| 1,3-Butylene glycol | 4.0 | 4.0 | 4.0 |
| 10% (w/v) Potassium hydroxide | 0.6 | 0.6 | 0.6 |
| Methylparaben | 0.1 | 0.1 | 0.1 |
| L-carnitine 2-hexyldecanoate hydrochloride | 0.01 | 5.0 | 1.0 |
| Cocoyl amide propyldimethyl glycine | 0.05 | | |
| Lauryl dimethylaminoacetic acid betaine | | 0.5 | |
| Lauric acid amide betaine propylacetate | | | 1.0 |
| Purified water | Balance | Balance | Balance |

| Conditions | | | |
|---|---|---|---|
| Immediately after preparation | Homogeneous Transparent | Homogeneous Transparent | Homogeneous Transparent |
| 24 Hours after preparation | Homogeneous Transparent | Homogeneous Transparent | Homogeneous Transparent |
| 1 Month after preparation | Homogeneous Transparent | Homogeneous Transparent | Homogeneous Transparent |

| | | | |
|---|---|---|---|
| * The values are weight percentages relative to the total weight at 100. | | | |

### [Example 1] pH dependency of formulation stability

Cosmetics having a pH in the range of 5 to 10 were prepared according to the formulation for the cosmetic 2 in Table 1 while adjusting the concentration of potassium hydroxide (10% in cosmetic 2). For reference, the pH of the cosmetic 2 was 7.0.

These cosmetics were dispensed in glass vials, and the glass vials were tightly closed and placed in an incubator at 25°C. The conditions of the cosmetics were visually observed immediately after the preparation, after 24 hours of standing, after 1 month of standing, and after 3 months of standing. The results are set forth in Table 5.

### [Table 5]

**Table 5**

| pH of cosmetics | Conditions of cosmetics | | | |
|---|---|---|---|---|
| | Immediately after preparation | After 24 hours | After 1 month | After 3 months |
| 5 | White turbid | Separated | (Not observed) | (Not observed) |
| 5.5 | Homogeneous Transparent | Homogeneous Transparent | Homogeneous Transparent | White turbid |
| 6 | Homogeneous Transparent | Homogeneous Transparent | Homogeneous Transparent | Homogeneous Transparent |
| 6.5 | Homogeneous Transparent | Homogeneous Transparent | Homogeneous Transparent | Homogeneous Transparent |
| 7 (cosmetic 2) | Homogeneous Transparent | Homogeneous Transparent | Homogeneous Transparent | Homogeneous Transparent |
| 7.5 | Homogeneous Transparent | Homogeneous Transparent | Homogeneous Transparent | Homogeneous Transparent |
| 8 | Homogeneous Transparent | Homogeneous Transparent | Homogeneous Transparent | Homogeneous Transparent |
| 8.5 | Homogeneous Transparent | Homogeneous Transparent | Homogeneous Transparent | White turbid |
| 9 | Homogeneous Transparent | Homogeneous Transparent | Homogeneous Transparent | White turbid |
| 9.5 | Homogeneous Transparent | White turbid | Separated | (Not observed) |
| 10 | White turbid | White turbid | Separated | (Not observed) |

### [Example 2] pH dependency of formulation stability

Cosmetics having a pH in the range of 5 to 10 were prepared according to the formulation for the cosmetic 6 in Table 2 while adjusting the concentration of potassium hydroxide (10% in cosmetic 6). For reference, the pH of the cosmetic 6 was 7.0.

These cosmetics were dispensed in glass vials, and the glass vials were tightly closed and placed in an incubator at 25°C. The conditions of the cosmetics were visually observed immediately after the preparation, after 24 hours of standing, after 1 month of standing, and after 3 months of standing. The results are set forth in Table 6.

### [Table 6]

**Table 6**

| pH of cosmetics | Conditions of cosmetics | | | |
|---|---|---|---|---|
| | Immediately after preparation | After 24 hours | After 1 month | After 3 months |
| 5 | White turbid | Precipitated | (Not observed) | (Not observed) |
| 5.5 | Homogeneous Transparent | Homogeneous Transparent | Homogeneous Transparent | White turbid |
| 6 | Homogeneous Transparent | Homogeneous Transparent | Homogeneous Transparent | Homogeneous Transparent |
| 6.5 | Homogeneous Transparent | Homogeneous Transparent | Homogeneous Transparent | Homogeneous Transparent |
| 7 (cosmetic 6) | Homogeneous Transparent | Homogeneous Transparent | Homogeneous Transparent | Homogeneous Transparent |
| 7.5 | Homogeneous Transparent | Homogeneous Transparent | Homogeneous Transparent | Homogeneous Transparent |
| 8 | Homogeneous Transparent | Homogeneous Transparent | Homogeneous Transparent | Homogeneous Transparent |
| 8.5 | Homogeneous Transparent | Homogeneous Transparent | Homogeneous Transparent | White turbid |
| 9 | Homogeneous Transparent | Homogeneous Transparent | Homogeneous Transparent | White turbid |
| 9.5 | White turbid | White turbid | Precipitated | (Not observed) |
| 10 | White turbid | Precipitated | (Not observed) | (Not observed) |

## Claims

1. A skin external preparation comprising a carnitine derivative represented by the following Formula (1) and/or a carnitine derivative salt represented by the following Formula (2), and an amphoteric surfactant; 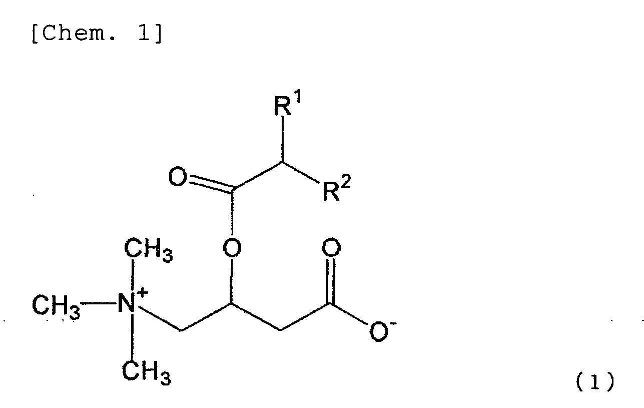 wherein R¹ and R² are each independently a C1-18 optionally branched, saturated or unsaturated aliphatic hydrocarbon group; 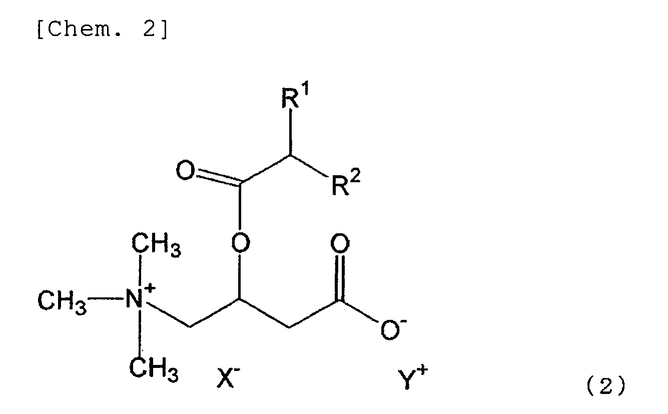 wherein R¹ and R² are the same as R¹ and R² in Formula (1), X⁻ is an inorganic or organic anion providing electrical neutrality with the cation part of the carnitine derivative, and Y⁺ is an inorganic or organic cation providing electrical neutrality with the anion part of the carnitine derivative.

2. The skin external preparation according to claim 1, which contains the carnitine derivative and/or the salt thereof at 0.01 to 5% by mass and the amphoteric surfactant in an amount that is 1/10 to 5 times the amount of the carnitine derivative and/or the salt thereof.

3. The skin external preparation according to claim 1 or 2, wherein R¹ and R² in Formulae (1) and (2) are each independently a C3-16 optionally branched, saturated or unsaturated aliphatic hydrocarbon group.

4. The skin external preparation according to any one of claims 1 to 3, wherein either of R¹ and R² in Formulae (1) and (2) is a C6 linear alkyl group and the other is a C8 linear alkyl group.

5. The skin external preparation according to any one of claims 1 to 4, wherein X- in Formula (2) is selected from the group consisting of hydroxide ion, nitrate ion, sulfate ion, carbonate ion, hydrogen carbonate ion, halide ion, formate ion, acetate ion, citrate ion, tartrate ion, oxalate ion, fumarate ion, C3-20 optionally branched, saturated or unsaturated fatty acid anion, carnitine anion, carnitine derivative anion, ascorbate anion, ascorbylphosphate anion and ascorbylphosphate derivative anion.

6. The skin external preparation according to any one of claims 1 to 5, wherein Y⁺ in Formula (2) is selected from the group consisting of hydrogen ion, sodium ion, potassium ion, calcium ion, magnesium ion, zinc ion, ammonium ion, carnitine cation and carnitine derivative cation.

7. The skin external preparation according to any one of claims 1 to 6, wherein the amphoteric surfactant is a betaine surfactant.

8. The skin external preparation according to claim 7, wherein the betaine surfactant is an aminoacetic acid betaine surfactant.

9. The skin external preparation according to claim 7, wherein the betaine surfactant is an alkyldimethylaminoacetic acid betaine or a fatty acid amidopropyldimethylaminoacetic acid betaine.

10. The skin external preparation according to claim 7, wherein the betaine surfactant is selected from the group consisting of lauryl dimethylaminoacetic acid betaine, cocoalkyl betaine, stearyl dimethyl amino acetic acid betaine, cocoyl amide propyl betaine, palm kernel oil amide propyl betaine, lauric acid amide betaine propylacetate, propyl betaine ricinoleate amide and stearyl dihydroxyethyl betaine.

11. The skin external preparation according to any one of claims 1 to 10, wherein the preparation has a pH in the range of 5.5 to 9.

12. The skin external preparation according to any one of claims 1 to 10, wherein the preparation has a pH in the range of 6.0 to 8.0.

13. The skin external preparation according to any one of claims 1 to 12, which is used as a cosmetic.
